# EUROPEAN PATENT APPLICATION

(11) **EP 3 162 282 A1**
(43) Date of publication of application: **03.05.2017**
(21) Application number: 15815610.9
(22) Date of filing: 29.06.2015
(51) Int. Cl.: A61B 3/10

(54) **TOMOGRAPHY DEVICE**

(30) Priority: 30.06.2014 JP 2014134108
(71) Applicant: KOWA Co., Ltd., Nagoya-shi, Aichi-ken 460-8625 (JP)
(72) Inventor: KOBAYASHI, Naoki, Higashimurayama-shi Tokyo 189-0022 (JP); KAKUTANI, Yoshihiro, Higashimurayama-shi Tokyo 189-0022 (JP)
(74) Representative: Lorenz & Kopf PartG mbB Patentanwälte, LKGLOBAL
(86) International application number: PCT/JP2015/068629
(87) International publication number: WO 2016/002693

(57) **Abstract**

A measurement optical system (30) is provided which causes measurement light split by a beam splitter (20) to be incident to an object, and a reference optical system (40) is provided which causes reference light split by the beam splitter to be incident to a reference mirror (49). A tomographic image of the object is formed on the basis of interference light generated by superposition of the measurement light reflected at the object to return and the reference light reflected at the reference object to return. Optical components such as lenses and mirrors that constitute the measurement optical system and reference optical system correspond to each other, and the wavelength dispersion characteristics of the optical components in the correspondence relationship are identical or equivalent.

## Description

### Technical Field

The present invention relates to a tomographic image capturing device that captures a tomographic image of an object such as a subject's eye on the basis of interference light generated by superposition of measurement light from the object and reference light.

### Background Art

As a type of ophthalmic diagnostic equipment, there are tomographic image capturing devices that utilize optical interference of so-called OCT (Optical Coherence Tomography) to capture tomographic images of ocular fundi. Such tomographic image capturing devices can capture tomographic images of ocular fundi at high resolution through irradiating the ocular fundi with broadband and low-coherent light and causing the reflected light from the ocular fundi to interfere with reference light.

In such a tomographic image capturing device, when an optical glass is inserted in either one of a measurement optical system and a reference optical system, the phase of light at the inserted side becomes delayed by an amount corresponding to the optical distance determined by the thickness and refractive index of the glass material, and this delay causes different phase delay amounts at different wavelengths depending on the refractive index dispersion of the optical glass. If the dispersion is zero, a signal from one reflecting surface will have an interference spectrum of a period corresponding to the phase delay amount. However, when subjected to some refractive index dispersion, the signal undergoes different phase delays at the short-wavelength side and long-wavelength side thereby to cause chirping of the period of interference spectrum, which may lead to a blurred image as the obtained tomographic image.

To compensate for such a phase delay in the optical paths of the measurement optical system and reference optical system due to insertion of an optical glass, a dispersion compensation glass is inserted in the reference optical path side in Patent Literature 1 and 2 below, and variable-thickness optical materials with different dispersion characteristics are disposed in the measurement light optical path or the reference light optical path in Patent Literature 3 below.

### Prior Art Literature

### Patent Literature

[Patent Literature 1] JP2010-169502A
[Patent Literature 2] JP2009-103688A
[Patent Literature 3] JP2008-501118A

### Summary of the Invention

### Problems to be solved by the Invention

Thus, according to the prior art, one or more dispersion compensation elements are expected to play a role to compensate for a difference in the refractive index dispersion characteristics of individual optical components, such as lenses and mirrors, which are disposed in the measurement optical system and the reference optical system. Therefore, the difference in the refractive index dispersion characteristics of individual optical components cannot be completely compensated for. In particular, compensation of higher-order dispersion is difficult for broadband light and the captured tomographic image tends to blur due to the effect of the refractive index dispersion, which may be problematic.

In addition to such refractive index dispersion, optical components involve phase dispersion due to a phase difference, which may also cause a blurred tomographic image. Wavelength dispersion refers to those including both the refractive index dispersion and the phase dispersion, and compensation of the wavelength dispersion plays an important role in obtaining a clear tomographic image.

The present invention has been made to solve the above problems and an object of the present invention is to provide a tomographic image capturing device that can capture a clear tomographic image by compensating for wavelength dispersion of optical components disposed in a measurement optical system and reference optical system.

### Means for solving the Problems

The present invention, which achieves the above object, comprises:
a splitting optical element configured to sprit light output from a light source into measurement light and reference light;
a measurement optical system configured to cause the measurement light split by the splitting optical element to be incident to an object;
a reference optical system configured to cause the reference light split by the splitting optical element to be incident to a reference object; and
a tomographic image forming means configured to form a tomographic image of the object on a basis of interference light generated by superposition of the measurement light reflected at the object to return via the measurement optical system and the reference light reflected at the reference object to return via the reference optical system,
wherein the reference optical system is composed of optical components corresponding to respective optical components that constitute the measurement optical system,
wherein wavelength dispersion characteristics of each optical component of the measurement optical system and each optical component of the reference optical system in a correspondence relationship are identical or equivalent.

The reference optical system may be provided with a dispersion compensation optical element for compensating for wavelength dispersion of the object. This dispersion compensation optical element may comprise a mechanism in which a plurality of optical elements is selectively inserted and removed and may be constituted as one or more turrets that comprise a plurality of glasses having different thicknesses and/or materials and are configured such that any of the glasses can be selected for combination.

When the measurement optical system is provided with a dichroic mirror, the reference optical system may be provided with a dichroic mirror having the same wavelength dispersion characteristics as those of the dichroic mirror of the measurement optical system so that the incident angle and the like are identical with those of the dichroic mirror of the measurement optical system.

### Advantageous Effect of the Invention

According to the present invention, the reference optical system is configured such that optical components corresponding to respective optical components that constitute the measurement optical system are disposed, and each optical component of the measurement optical system and each optical component of the reference optical system in a correspondence relationship are identical or equivalent with respect to the wavelength dispersion characteristics. This can avoid the occurrence of a phase difference that would require dispersion compensation for both the optical systems. Therefore, with regard to the optical systems, an effect is obtained that a clear tomographic image can be acquired without separately requiring an additional optical element for dispersion compensation in the reference optical system or in the measurement optical system.

Moreover, the dispersion compensation optical element for compensating for the wavelength dispersion of an object may be disposed in the reference optical system and it is therefore possible to obtain a clear tomographic image of the object in which the wavelength dispersion of the object is compensated for. In this case, one can concentrate on compensating for the wavelength dispersion of the object and can effectively perform the dispersion compensation depending on the object because respective corresponding optical components of the measurement optical system and the reference optical system are identical or equivalent with respect to the wavelength dispersion characteristics and the dispersion compensation is not necessary.

### Brief Description of Drawings

FIG. 1 is an optics view illustrating the overall configuration of an example of a tomographic image capturing device.
FIG. 2 is an optics perspective view illustrating details of a reference optical system of FIG. 1.
FIG. 3 is an optics view illustrating the overall configuration of another example of a tomographic image capturing device.
FIG. 4 is an optics perspective view illustrating details of a reference optical system of FIG. 3.
FIG. 5 is a front elevational view of a dispersion compensation glass turret.
FIG. 6 is an optics view illustrating a modified example of a reference optical system.
FIG. 7 is an optics view illustrating another modified example of a reference optical system.

### Embodiments for Carrying out the Invention

Hereinafter, the present invention will be described in detail with reference to examples illustrated in the drawings.

### Example 1

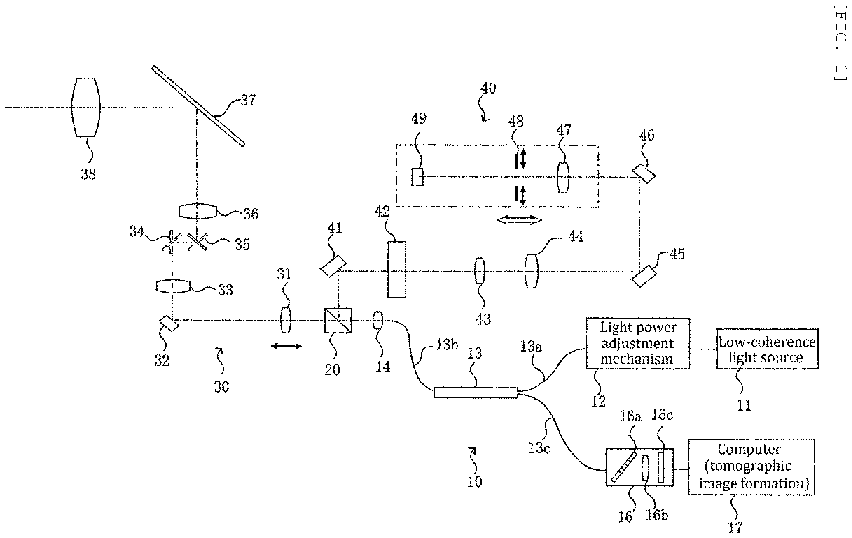

FIG. 1 illustrates optical systems of a tomographic image capturing device as a whole. The part denoted by reference numeral 10 is a demultiplexing/multiplexing optical system. This optical system is provided with a broadband low-coherence light source 11 that comprises, for example, a super-luminescent diode (SLD) and emits light of a temporal coherence length of about several micrometers to several tens of micrometers at a wavelength of 700 nm to 1,100 nm.

The low-coherence light generated from the low-coherence light source 11 passes through a light power adjustment mechanism 12, in which the light power is adjusted, and is incident to an optical coupler 13 via an optical fiber 13a and then introduced into a beam splitter 20 as a splitting optical element via an optical fiber 13b and collimator lens 14. Demultiplexing and/or multiplexing may also be performed using an optical circulator as substitute for the optical coupler 13.

The light incident to the beam splitter 20 is split into reference light and measurement light. The measurement light is incident to a focusing lens 31, which is to focus the measurement light on an object (not illustrated). The measurement light to be focused on the object is reflected by a mirror 32, passes through a lens 33, and is scanned in an arbitrary direction by an x-axis scanning mirror (galvanometer mirror) 34 and y-axis scanning mirror (galvanometer mirror) 35. The measurement light scanned by the x-axis and y-axis scanning mirrors 34 and 35 passes through a scanning lens 36, is reflected by a dichroic mirror 37, and then passes through an objective lens 38 to be incident to the object, which is thus scanned by the measurement light in the x-direction and y-direction. The measurement light reflected by the object tracks back the above path to return to the beam splitter 20.

In such an optical system, the focusing lens 31, mirror 32, lens 33, x-axis scanning mirror 34, y-axis scanning mirror 35, scanning lens 36, dichroic mirror 37 and objective lens 38, which are located downstream the beam splitter 20, constitute a measurement optical system 30 of the tomographic image capturing device.

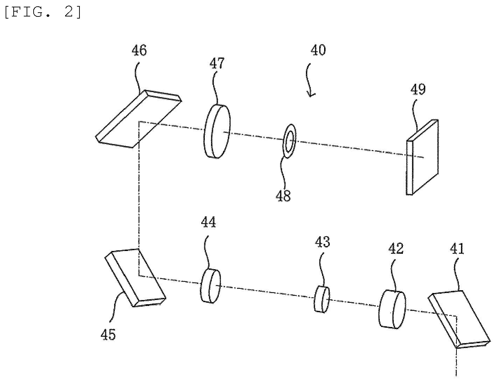

On the other hand, the reference light split by the beam splitter 20, as illustrated in detail in FIG. 2, is reflected by a mirror 41, then passes through a dispersion compensation glass for objective lens 42 and lenses 43 and 44, is thereafter reflected by a mirror 45 and dichroic mirror 46, passes through a focusing lens 47 and variable aperture stop 48, and reaches a reference mirror 49. The variable aperture stop 48 has a variable aperture diameter to adjust the light power. To adjust the optical path length, the focusing lens 47, variable aperture stop 48 and reference mirror 49 can move in the optical axis direction in an integrated manner, as indicated by the blank arrow in FIG. 1. The reference light reflected by the reference mirror 49 tracks back the above path to return to the beam splitter 20.

In such an optical system, the mirror 41, dispersion compensation glass for objective lens 42, lenses 43 and 44, mirror 45, dichroic mirror 46, focusing lens 47 and reference mirror 49 constitute a reference optical system 40 of the tomographic image capturing device. The reference mirror 49 acts as a reference object.

The measurement light and reference light returned to the beam splitter 20 are superposed with each other to be interference light, which passes through the collimator lens 14 and optical coupler 13 and is incident to a spectroscope 16 via an optical fiber 13c. The spectroscope 16 has a diffraction grating 16a, imaging lens 16b, line sensor 16c, and other necessary components. The interference light is diffracted by the diffraction grating 16a into a spectrum in accordance with the wavelength of the low-coherence light and forms an image on the line sensor 16c by the imaging lens 16b.

Signals from the line sensor 16c are subjected to signal processing, including Fourier transformation, performed by a tomographic image forming means that is realized using one or more CPUs of a computer 17 and the like. This signal processing generates a depth signal that represents information in the depth direction (z-direction) of the object. When scanning the object, the interference light at each sampling time point allows the depth signal (A-scan picture) at the sampling time point to be obtained. Therefore, completion of one scanning can form a two-dimensional tomographic image (B-scan picture) that comprises a z-direction picture (A-scan picture) along the scanning direction.

In such a tomographic image capturing device, various optical components such as lenses and mirrors are disposed in the measurement light optical path of the measurement optical system 30 and the reference light optical path of the reference optical system 40. These optical components may have different wavelength dispersion characteristics. Such a difference in the wavelength dispersion characteristics of optical components causes different phase delay amounts at different wavelengths and the chirping occurs in the period of interference spectrum obtained from the spectroscope 16. The Fourier-transformed tomographic image will thus be a blurred tomographic image.

Such a problem is solved by the present invention in which each optical component that constitutes the measurement optical system and each optical component that constitutes the reference optical system are identical or equivalent components with respect to the wavelength dispersion characteristics so that the respective optical components disposed in the measurement optical system and reference optical system are symmetric.

First, in the optical components such as lenses in which light passes through, refractive index dispersion among wavelength dispersions occurs in accordance with the thickness and refractive index of a glass material of each lens. Therefore, the reference optical system 40 uses a lens or optical glass that has identical or equivalent dispersion characteristics to those of each lens of the measurement optical system 30. For example, for the objective lens 38 of the measurement optical system 30, the dispersion compensation glass for objective lens 42 which has identical or equivalent refractive index dispersion characteristics to those of the objective lens 38 is disposed in the reference optical path of the reference optical system 40. Similarly, for the scanning lens 36 of the measurement optical system 30, the focusing lens 47 which has identical or equivalent refractive index dispersion characteristics to those of the scanning lens 36 is used in the reference optical system 40. For the lens 33 of the measurement optical system 30, the lens 44 which has identical or equivalent refractive index dispersion characteristics to those of the lens 33 is used in the reference optical system 40. For the focusing lens 31 of the measurement optical system 30, the lens 43 which has identical or equivalent refractive index dispersion characteristics to those of the focusing lens 31 is used.

For simplicity, FIG. 1 and FIG. 2 illustrate each lens as a single lens, but when a lens of the measurement optical system is a compound lens that is composed of a plurality of lenses, the compound lens may be regarded as one lens and the reference optical system can use a single lens or compound lens that has identical or equivalent refractive index dispersion characteristics to those of that one lens.

The scanning lens 36 of the measurement optical system 30 acts to determine the scanning range while the focusing lens 47 of the reference optical system 40 corresponding to the scanning lens 36 has a function to converge light. They thus have different functions, but the refractive index dispersion characteristics are identical or equivalent. Therefore, insertion of these lenses 36 and 47 into the measurement optical system and reference optical system can avoid the occurrence of a phase difference that would require dispersion compensation for both the optical systems. Similarly, the objective lens 38 and the dispersion compensation glass for objective lens 42 corresponding thereto, the lens 33 and the lens 44 corresponding thereto, and the focusing lens 31 and the lens 43 corresponding thereto, have different functions in the measurement optical system 30 and in the reference optical system 40, but the refractive index dispersion characteristics are identical or equivalent. Therefore, insertion of the corresponding lenses into the measurement optical system and reference optical system can avoid the occurrence of a phase difference that would require dispersion compensation for both the optical systems.

In the optical components such as mirrors at which light is reflected, phase dispersion among wavelength dispersions occurs. However, the reference optical system 40 uses the dichroic mirror 46 of which the phase dispersion characteristics are identical or equivalent to those of the dichroic mirror 37 of the measurement optical system 30, and the mirror 32, x-axis scanning mirror 34 and y-axis scanning mirror 35 of the measurement optical system 30 and the mirror 41, mirror 45 and reference mirror 49 of the reference optical system 40 are respectively identical or equivalent with respect to the phase dispersion characteristics. Therefore, the use of respective mirrors in the measurement optical system 30 and reference optical system 40 does not generate a phase difference that would require dispersion compensation.

An ND filer may be used for adjustment of the light power in the reference optical system, but in the present example, the variable aperture stop 48 having a variable aperture diameter is used because it is preferred to use a light power adjustment mechanism that can adjust the light power without generation of dispersion so that a difference in the refractive index dispersion does not occur between the measurement optical system and the reference optical system. The location at which the variable aperture stop 48 is disposed is not limited to the location illustrated in FIG. 1 and the variable aperture stop 48 can be disposed at any other part of the optical path of the reference optical system 40.

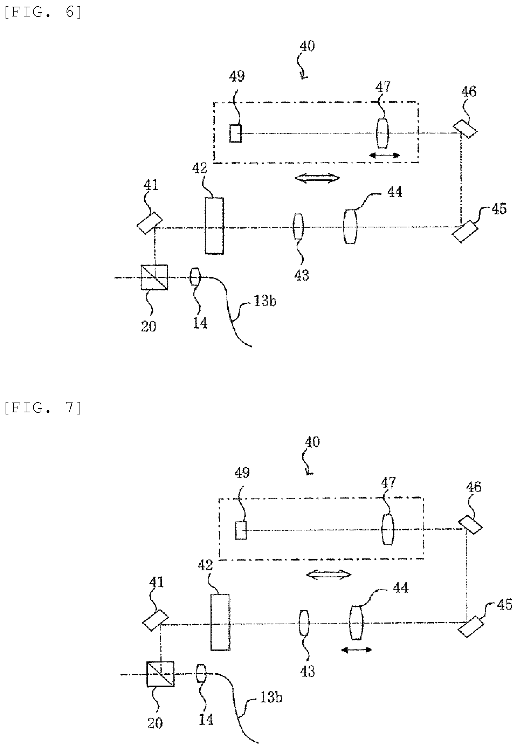

To adjust the light power without generating refractive index dispersion, it is also possible to employ a method in which the distance between the focusing lens 47 and the reference mirror 49 is made variable. This example is illustrated in FIG. 6, in which the focusing lens 47 is moved along the optical axis to vary the distance between the focusing lens 47 and the reference mirror 49 thereby to adjust the light power in the reference optical system 40.

Instead of varying the distance between the focusing lens 47 and the reference mirror 49, varying the distance between the lens 43 and the lens 44 also allows the light power to be adjusted in the reference optical system 40. This example is illustrated in FIG. 7, in which the lens 44 is moved in the optical axis direction to vary the distance between the lens 43 and the lens 44 thereby to adjust the light power in the reference optical system 40. In FIG. 7, the lens 44 is moved to vary the distance between the lens 43 and the lens 44, but the lens 43 may be moved to vary the distance between the lenses 43 and 44.

Thus, in the present example, the reference optical system 40 is configured such that optical components corresponding to respective optical components that constitute the measurement optical system 30 are disposed, and each optical component of the measurement optical system 30 and each optical component of the reference optical system 40 in a correspondence relationship are identical or equivalent with respect to the wavelength dispersion characteristics. This can avoid the occurrence of a phase difference that would require dispersion compensation for both the optical systems. Therefore, a clear tomographic image can be acquired without separately requiring an additional optical element for dispersion compensation in the reference optical system or in the measurement optical system.

When the dichroic mirror 37 is used in the measurement optical system 30 and its phase distribution exhibits steep variation rather than moderate variation, different phase changes may occur at different wavelengths and the pattern of interference signal may be disturbed to make the tomographic image blurred. In addition, different phase changes may occur at different polarization directions of the incident light.

In the present example, such phase changes can be canceled because the reference optical system 40 uses the same dichroic mirror 46 as the dichroic mirror 37 of the measurement optical system 30. Note, however, that the phase changes depend on the incident angle. Accordingly, when the dichroic mirror 37 is disposed downstream the scanning mirrors 34 and 35 as illustrated in FIG. 1, scanning by the scanning mirrors 34 and 35 may vary the incident angle to the dichroic mirror 37 to change the reflection phase characteristics. Therefore, in the case of a dichroic mirror having a part at which the phase distribution exhibits steep variation, the phase changes may not be completely compensated for even when the same dichroic mirrors are used in the measurement optical system 30 and in the reference optical system 40. However, when the phase changes are moderate, the phase changes can be compensated for by using the dichroic mirrors 37 and 46 having the same or equivalent phase dispersion characteristics in the measurement optical system and the reference optical system, respectively, as illustrated in FIG. 1. Furthermore, when the dichroic mirror 46 of the reference optical system is disposed at such a position that allows the incident angle to be the same as that to the dichroic mirror 37 of the measurement optical system, the phase changes can be further well compensated for.

When the dichroic mirror 37 is a dichroic mirror for which the phase dispersion compensation is performed, it is not necessary to provide a mirror corresponding to the dichroic mirror 37 of the measurement optical system because the influence of the phase dispersion can be almost neglected. In this case, therefore, an ordinary total-reflection metal mirror can be used.

In the present example, as illustrated in FIG. 1, the demultiplexing/multiplexing optical system 10 using an optical fiber or optical circulator is disposed between the light source 11 and the beam splitter 20 which splits the light from the light source 11 into the measurement light and the reference light. In an ordinary scheme of using an optical fiber for split into the measurement light and reference light, the use of optical fiber undergoes the influences of mode dispersion and wavelength dispersion. In the present example, however, the optical fiber is provided in the demultiplexing/multiplexing optical system and no optical fiber is used in the measurement optical system and the reference optical system. Therefore, an advantage can be obtained that the influences of mode dispersion and wavelength dispersion of an optical fiber may not necessarily be required to be compensated for in the reference optical system or in the measurement optical system.

### Example 2

In the above-described Example 1, the reference optical system employs the same optical components as those used in the measurement optical system with respect to the wavelength dispersion characteristics including refractive index dispersion and phase dispersion. Therefore, almost complete wavelength dispersion compensation can be realized.

However, when an object such as a subject's eye having refractive index dispersion is disposed in the measurement optical system, a phase difference due to the refractive index dispersion of the object may occur between the measurement optical system and the reference optical system even though the wavelength dispersions in the corresponding optical components of the measurement optical system and reference optical system are identical or equivalent. If such a phase difference is not compensated for, the obtained tomographic image will be a blurred image.

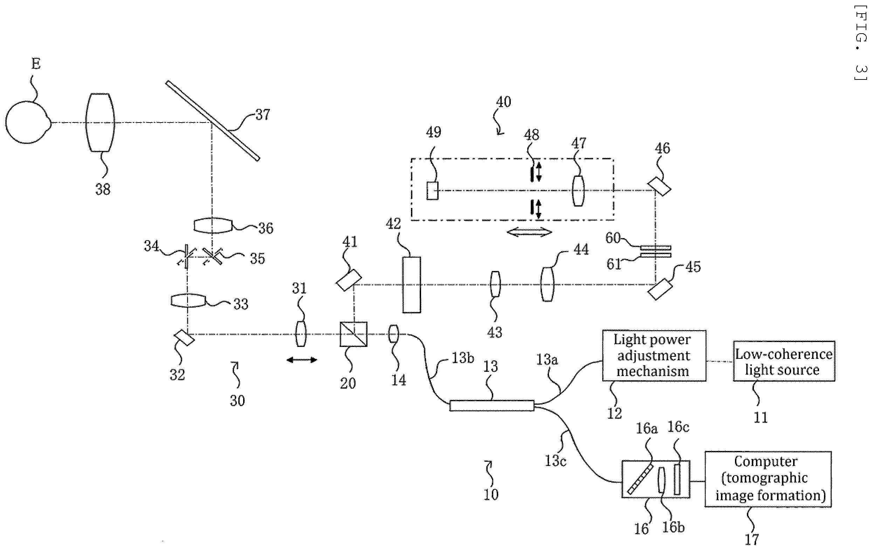

In this regard, FIG. 3 to FIG. 5 illustrate Example 2 in which the object is a human eye and its refractive index dispersion is compensated for. In the description below, the same or similar elements, components and devices as in Example 1 are denoted by the same reference numerals and the description thereof will be omitted.

In FIG. 3, a dispersion compensation optical element is disposed in the reference optical system in order to compensate for the refractive index dispersion of a subject's eye E. This dispersion compensation optical element is composed of two turrets 60 and 61 that are each formed with a plurality of openings at regular intervals along the circumference. One of the openings formed in each of the turrets 60 and 61 is a thorough-hole, another one is a closure plate, and other openings are provided with glasses having different thicknesses and/or materials.

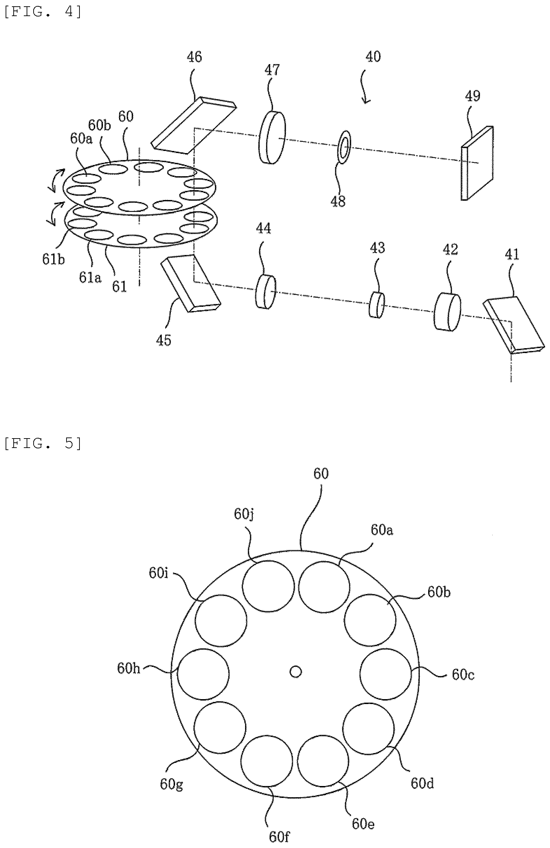

As illustrated in FIG. 5, the turret 60 is provided with glasses 60a to 60h, for example, of the same glass materials but different thicknesses, a closure plate 60i and a thorough-hole 60 j, any of which can be inserted into the optical path of the reference optical system. Similarly, the turret 61 is provided with glasses 61a, 61b, ..., a closure plate and a through-hole, any of which can be inserted into the optical path of the reference optical system. In the turret 61, the glasses 61a, 61b, ... are made of glass materials that are different from those used in the turret 60, and the thicknesses thereof are different. These turrets 60 and 61 are rotated to insert any glass of each of the turrets 60 and 61 into the optical path of the reference optical system 40 and various combinations of dispersion compensation glasses can thereby be obtained.

An optimum combination of dispersion compensation glasses of the turrets 60 and 61 are determined in the following manner. First, the focusing lens 31 is moved to perform focus adjustment in accordance with a diopter scale of the subject's eye and the obtained tomographic image is displayed on a display (not illustrated) connected to the computer 17, to determine a correction amount for the diopter scale of the subject's eye. Then, dispersion compensation glasses expected to be optimum for the diopter scale are selected from the combinations of glasses of the turrets 60 and 61 and the combination is stored as an initial value. Thereafter, the compensation amount for dispersion is varied while rotating the turrets 60 and 61, and the optimum combination of compensation amounts is automatically determined such that a value of image quality parameter for evaluating the image quality of the captured tomography image becomes largest. Through this operation, the refractive index dispersion of the subject's eye can be optimally compensated for.

When the object is a subject's eye, the ocular fundus of the subject's eye can be focused on to capture a tomographic image of the ocular fundus and the anterior eye part can also be focused on to capture a tomographic image of the anterior eye part.

As will be understood, also in Example 2, the configurations as illustrated in FIG. 6 and FIG. 7 may be used to adjust the light power in the reference optical system 40.

Thus, in Example 2, the dispersion compensation optical element for compensating for the refractive index dispersion of an object is disposed in the reference optical system and it is therefore possible to obtain a clear tomographic image of the object in which the refractive index dispersion of the object is compensated for. In this case, one can concentrate on compensating for the refractive index dispersion of the object and can effectively perform the dispersion compensation depending on the object because each optical component that constitutes the measurement optical system and the corresponding optical component of the reference optical system are identical or equivalent with respect to the wavelength dispersion characteristics and the dispersion compensation due to the arrangement of respective corresponding optical components is not necessary.

### Description of Reference Numerals:

- 10: Demultiplexing/multiplexing optical system
- 11: Low-coherence light source
- 12: Light power adjustment mechanism
- 13: Optical coupler
- 14: Collimator lens
- 20: Beam splitter
- 30: Measurement optical system
- 31: Focusing lens
- 34: X-axis scanning mirror
- 35: Y-axis scanning mirror
- 36: Scanning lens
- 37: Dichroic mirror
- 38: Objective lens
- 40: Reference optical system
- 42: Dispersion compensation glass for objective lens
- 46: Dichroic mirror
- 47: Focusing lens
- 48: Variable aperture stop
- 49: Reference mirror
- 60, 61: Turret

## Claims

1. A tomographic image capturing device comprising:
a splitting optical element configured to sprit light output from a light source into measurement light and reference light;
a measurement optical system configured to cause the measurement light split by the splitting optical element to be incident to an object;
a reference optical system configured to cause the reference light split by the splitting optical element to be incident to a reference object; and
a tomographic image forming means configured to form a tomographic image of the object on a basis of interference light generated by superposition of the measurement light reflected at the object to return via the measurement optical system and the reference light reflected at the reference object to return via the reference optical system,
wherein the reference optical system is composed of optical components corresponding to respective optical components that constitute the measurement optical system,
wherein wavelength dispersion characteristics of each optical component of the measurement optical system and each optical component of the reference optical system in a correspondence relationship are identical or equivalent.

2. The tomographic image capturing device as recited in claim 1, wherein the reference optical system is provided with a dispersion compensation optical element for compensating for wavelength dispersion of the object.

3. The tomographic image capturing device as recited in claim 2, wherein the dispersion compensation optical element comprises a mechanism in which a plurality of optical elements is selectively inserted and removed.

4. The tomographic image capturing device as recited in claim 3, wherein the mechanism in which the plurality of optical elements is selectively inserted and removed includes one or more turrets that comprise a plurality of glasses having different thicknesses and/or materials and are configured such that any of the glasses can be selected for combination.

5. The tomographic image capturing device as recited in any one of claims 1 to 4, wherein the measurement optical system is provided with a dichroic mirror and the reference optical system is provided with a dichroic mirror of which dispersion characteristics are identical or equivalent to those of the dichroic mirror of the measurement optical system.

6. The tomographic image capturing device as recited in claim 5, wherein the dichroic mirror of the reference optical system is disposed at a position at which an incident angle to the dichroic mirror is same as an incident angle to the dichroic mirror of the measurement optical system.

7. The tomographic image capturing device as recited in any one of claims 1 to 6, wherein the reference optical system is provided with a light power adjustment mechanism configured to be able to adjust a light power without generating dispersion so that a difference in refractive index dispersion does not occur between the measurement optical system and the reference optical system.

8. The tomographic image capturing device as recited in claim 7, wherein the light power adjustment mechanism comprises a variable aperture stop having a variable aperture diameter.

9. The tomographic image capturing device as recited in claim 7, wherein the light power adjustment mechanism moves a position of a lens that constitutes the reference optical system in an optical axis direction thereby to adjust the light power in the reference optical system.

10. The tomographic image capturing device as recited in any one of claims 1 to 9, further comprising
a demultiplexing/multiplexing optical system configured to demultiplex and/or multiplex the light from the light source, the demultiplexing/multiplexing optical system being disposed between the light source and the splitting optical element.

11. The tomographic image capturing device as recited in claim 10, wherein the demultiplexing/multiplexing optical system comprises an optical fiber and an optical coupler or optical circulator configured to demultiplex and/or multiplex light introduced by the optical fiber.

12. The tomographic image capturing device as recited in any one of claims 1 to 11, wherein the object is a subject's eye.

13. The tomographic image capturing device as recited in claim 12, wherein an initial value of a compensation amount for refractive index dispersion of the object is determined in accordance with a diopter scale of the subject's eye.
